# EUROPÄISCHE PATENTSCHRIFT

(11) **EP 0 524 583 B1**
(45) Veröffentlichungstag und Bekanntmachung des Hinweises auf die Patenterteilung: **18.03.1998**
(21) Anmeldenummer: 92112384.0
(22) Anmeldetag: 20.07.1992
(51) Int. Cl.: G01N 33/53, G01N 33/68, G01N 33/563

(54) **Verfahren zur turbidimetrischen oder nephelometrischen Bestimmung von Analyten**
Procedure for the turbidimetric or nephelometric determination of analytes
Procédé pour la détermination turbidimétrique ou nephélométrique des analytes

(30) Priorität: 23.07.1991 DE 4124324
(43) Veröffentlichungstag der Anmeldung: 27.01.1993
(73) Patentinhaber: MERCK PATENT GmbH, D-64271 Darmstadt (DE)
(72) Erfinder: Dreher, Michael, W-6108 Weiterstadt (DE); Linxweiler, Winfried, W-6101 Messel (DE); Neumann, Siegfried, W-6104 Seeheim-Jugenheim (DE)

(56) Entgegenhaltungen:
- EP-A- 0 175 560
- EP-A- 0 347 138
- WO-A-89/00694
- PATENT ABSTRACTS OF JAPAN vol. 10, no. 177 (P-470)(2233) 21. Juni 1986
- ANNALS OF CLINICAL BIOCHEMISTRY Bd. 20, Nr. 1, Januar 1983, LONDON, GB Seiten 1 - 14 C. PRICE, K. SPENCER, AND J. WHICHER 'Light-scattering immunoassay of specific proteins: a review'

## Beschreibung

Die Erfindung betrifft Verfahren zur turbidimetrischen oder nephelometrischen Bestimmung von Analyten in Flüssigkeiten mit Hilfe einer Antikörper-Bindungsreaktion.

Sowohl die Immunturbidimetrie als auch die Immunnephelometrie basieren auf der Wechselwirkung zwischen Antikörpern und nachzuweisendem Antigen. Als Folge dieser Wechselwirkung entstehen hochmolekulare Aggregate, die als Streuzentren für einfallendes Licht wirken. Die Streuung des Lichts wird bei der Immunturbidimetrie als Anstieg der Extinktion, bei der Immunnephelometrie als Zunahme einer Streulichtintensität unter einem Winkel > 0° - ≤ 90° gegenüber dem einfallenden Licht registriert. Da das Ausmaß der Lichtstreuung ungefähr proportional 1/λ⁴ ist, werden für die Immunturbidimetrie und die Immunnephelometrie typischerweise kurzwellige Wellenlängenbereiche (320-360 nm) gewählt. Die theoretischen Grundlagen der Immunpräzipitation werden z.B. in Immunology 32, 445-457 (1977) beschrieben.

Die optimalen Bedingungen zur Durchführung solcher Bestimmungen wie Puffer, Detergentien, Neutralsalze, Beschleuniger usw. sind in der Literatur beschrieben und variieren geringfügig in Abhängigkeit von den jeweils verwendeten Antikörpern und den nachzuweisenden Antigenen (Ann. Clin. Biochem. 20, 1-14 (1983)).

Der große Vorteil der bisher beschriebenen Immunturbidimetrie und Immunnephelometrie liegt in der relativ einfachen Automatisierbarkeit der Verfahren. Die Immunturbidimetrie und die Immunnephelometrie sind für viele Serumproteine beschrieben (The Plasma Proteins, Vol. 2, Academic Press, New York, S. 375-425 (1975), Automated Immunoanalysis, Part 1+2, R.F. Ritchie (ed.), Marcel Dekker Inc., New York, Basel (1978)).

Die Nachweisgrenzen sind im Stand der Technik unterschiedlich dokumentiert und variieren in Abhängigkeit von Antikörper und Antigen. Die Nachweisgrenzen für die Immunturbidimetrie liegen bei ca. 5 mg/l, für die Immunnephelometrie bei ca. 1 mg/l. Die Meßbereichsobergrenzen für die Immun-turbidimetrie und die Immunnephelometrie liegen bei ca. 200-500 mg/l, je nach Testoptimierung.

Niedriger konzentrierte Analyte lassen sich mit der Immunturbidimetrie und der Immunnephelometrie nicht bestimmen. Hier setzt z.B. die Latexagglutination ein, die relativ große, je nach Testprinzip mit Antikörpern bzw. Antigenen beschichtete Partikel (ca. 0,05-3 µm Durchmesser) verwendet. Mit der Latexagglutinationstechnik lassen sich Nachweisgrenzen < 1 µg/l erreichen. Die typischen Meßbereichsobergrenzen liegen dann allerdings bereits bei 200-1000 µg/l (0,2-1 mg/l).

Der vorliegenden Erfindung liegt die Aufgabe zugrunde, Verfahren zur turbidimetrischen oder nephelometrischen Bestimmung von Analyten zur Verfügung zu stellen, die empfindlicher sind als die bekannten Verfahren und die den Nachweis von Antigenen im Konzentrationsbereich von 0,1 - > 100 mg/l erlauben.

Aus EP-A-0 347 138 ist z.B. bekannt, daß spezifische Bindungspartner in Mischung mit Proteinen zur Bestimmung von Liganden verwendet werden können. In EP-A-0 175 560 sind Kopplungsprodukte aus Enzympolymerisaten und einem Antikörper beschrieben, wobei die Bestimmung über die Messung der Enzymaktivität erfolgt. Weiter ist aus EP-A-0 142 301 eine elektrochemische Bestimmungsmethode beschrieben, wobei redox-modifizierte Kopplungsprodukte verwendet werden und die Störung der elektrochemischen Charakteristik der Komponenten als Meßgröße dient. Aus diesem Stand der Technik sind keine Hinweise auf turbidimetrische oder nephelometrische Bestimmungen zu entnehmen, noch sind dort Kopplungsprodukte aus mehreren Antikörpern oder deren Fragmenten an ein einziges Protein erwähnt.

Gegenstand der Erfindung ist ein Verfahren zur turbidimetrischen oder nephelometrischen Bestimmung von Analyten in Flüssigkeiten mit Hilfe einer Antikörper-Bindungsreaktion, das dadurch gekennzeichnet ist, daß man ein Polypeptid mit Antikörpern oder deren Fragmenten umsetzt, das daraus erhaltene Kopplungsprodukt mit dem Analyten inkubiert und die entstehende Trübung mißt.

Überraschenderweise wurde gefunden, daß polymerisierte Antikörper, die z. B. durch Säurefällung von Antiserum gewonnen wurden, im turbidimetrischen Test ein höheres Meßsignal liefern als monomere Antikörper. Allerdings schwankt der Polymerisationsgrad dieser Antikörper und damit das Meßsignal während der Lagerung. Zur Vermeidung dieses Nachteils wurde ein definierter, stabiler Komplex mit mehrfachen Bindungsstellen durch Kopplung von nativem IgG oder Antikörperfragmenten, vorzugsweise Fab'-Fragmenten, an einen molekularen Träger hergestellt.

Als molekulare Träger eignen sich Polypeptide, wie Proteine mit einem Molekulargewicht von mindestens 10 000 D. Vorzugsweise werden globuläre Proteine verwendet, wie Albumin, Thyroglobulin, Transferrin, Hemocyanin oder auch Polypeptide wie Polylysin. Ebenso kann das bekannte System Avidin/Biotin bzw. Streptavidin/Biotin eingesetzt werden, wobei biotinylierte Antikörper oder deren Fragmente einen stabilen Komplex mit Avidin bzw. Streptavidin bilden.

Die Kopplung an die Antikörper oder deren Fragmente kann auf verschiedene Arten durchgeführt werden. Vorzugsweise werden bifunktionelle Heterocrosslinker, z.B. Succinimidyl-Maleimid Crosslinker und verwandte Substanzen dafür eingesetzt. Der Vorteildieser Crosslinker liegt in der Vermeidung unerwünschter Kopplungsprodukte, der stufenweisen Einführung von Antikörpern bzw. Antikörperfragmenten, der end-on-Kopplung der Antikörper bzw. Antikörperfragmente und der chromatographisch leicht zu reinigenden Kopplungsprodukte.

Die Herstellung der Kopplungsprodukte erfolgt im allgemeinen über die folgenden Schritte:
1. Spaltung der Antikörper, z.B. mit Pepsin,
2. Chromatographische Reinigung der F(ab')₂-Fragmente,
3. Reduktion der F(ab')₂-Fragmente,
4. Chromatographische Isolierung der Fab'-Fragmente,
5. Kopplung des Crosslinkers an den Träger,
6. Chromatographische Reinigung des Träger-Crosslinker-Konjugats,
7. Kopplung der Fab'-Fragmente an das Träger-Crosslinker-Konjugat,
8. Chromatographische Reinigung des Kopplungsprodukts.

Das Träger-Crosslinker-Konjugat kann problemlos im Batchverfahren hergestellt und in Lösung eingefroren oder lyophilisiert aufbewahrt werden. Ebenso können die F(ab')₂-Fragmente im Batchverfahren hergestellt und wie oben beschrieben aufbewahrt werden. Für die eigentliche Herstellung werden dann nur die Schritte 3, 4, 7 und 8 benötigt.

Im Vergleich zu den bekannten Reagenzien ergeben die so hergestellten Reagenzien im turbidimetrischen und nephelomtrischen Test deutlich höhere Meßsignale und erlauben somit eine Steigerung der empfindlichkeit. Die Nachweisempfindlichkeit kann durch die Anwesenheit bekannter Acceleratoren(z.B. Polyethylenglycole) weiter optimiert werden (JP-A-61 25 062). Die so hergestellten Reagenzien sind in Lösung stabil, sie lassen sich problemlos auf Analysenautomaten einsetzen und eignen sich somit auch für die automatisierte immunturbidimetrische bestimmung.

Sowohl die immunturbidimetrische als auch die nephelometrische Bestimmung wird bei 320-400 nm, vorzugsweise bei etwa 340 nm durchgeführt. Die Bestimmung kann kinetisch, vorzugsweise jedoch als Endpunktreaktion, durchgeführt werden.

Mit dem Verfahren nach der vorliegenden Erfindung lassen sich alle Antigene, d.h. Substanzen, die über mehrere Bindungsstellen für Antikörper verfügen, bestimmen. Solche Eiweißkörper sind z.B. Transferrin, CRP, α₁-Mikroglobulin, RF, TBG, RBP, C3, C4, Ig's usw., die in Körperflüssigkeiten vorkommen und von diagnostischer Relevanz sind. Diese Eiweißkörper werden im Konzentrationsbereich von 0,05 - > 100 mg/l, vorzugsweise 0,5 - 100 mg/l, ohne weitere Vorverdünnung erfaßt. Haptene, die nur über eine Bindungsstelle verfügen, lassen sich kompetitiv unter Verwendung von sogenannten Polyhaptenen bestimmen.

### Beispiel 1

### Herstellung eines anti-Transferrin Fab'-Albumin-Konjugats

3,2 g anti-Transferrin-IgG vom Schaf werden in 150 ml 0,9 % NaCl-Lösung aufgenommen. Die Lösung wird mit 1 M Essigsäure auf pH = 4,5 eingestellt. Zu dieser Lösung werden 80 mg Pepsin zugegeben, gemischt und ca. 16 h bei 37 °C inkubiert. Die Reaktionslösung wird mit 1 M TRIS-Lösung auf pH = 7,0 eingestellt. Die Reaktionslösung wird affinitätschromatographisch mit einer Sepharose® 4B-Säule gereinigt, an die humanes Transferrin kovalent gebunden ist. Die gebundenen F(ab')₂-Fragmente werden mit 0,1 M Glycin/HCl (pH = 2,8) eluiert. Die proteinhaltigen Fraktionen werden gepoolt und mit 1 M TRIS-Lösung auf pH = 7,0 eingestellt. Die Lösung wird auf 30 ml in einer Ultrafiltrationszelle aufkonzentriert. Nach photometrischer Bestimmung werden 376 mg spezifische F(ab')₂-Fragmente erhalten.

16 ml dieser Lösung werden mit 1 M Essigsäure auf pH = 4,9 eingestellt, mit Argon begast und überschichtet und mit 0,9 ml einer 11%igen Lösung von Cysteaminhydrochlorid in Wasser versetzt. Nach 2 h Inkubaton bei 37 °C werden die Fab'-Fragmente gelchromatographisch über Sephadex® G-25 (150 mM NaCl, 20 mM Phosphatpuffer, pH = 7,0) gereinigt.

Die proteinhaltigen Fraktionen werden gepoolt. Nach photometrischer Bestimmung werden 190 mg Fab'-Fragmente in 34 ml Lösung erhalten. Die Lösung wird unter Argongas gekühlt aufbewahrt.

40,5 mg Rinderserumalbumin werden in 7 ml Wasser gelöst und mit 1 ml einer Lösung von 30 mg Succinimidyl-4-(N-Maleimidomethyl)cyclohexan-1-carboxylat (SMCC) in Dioxan versetzt.Die Reaktionslösung wird 1 h bei 30 °C inkubiert und anschließend gelchromatographisch über Sephadex® G-25 gereinigt. Die proteinhaltigen Fraktionen werden gepoolt. Nach photometrischer Bestimmung werden 38 mg des Kopplungsproduktes in 14,8 ml Lösung erhalten. Unter Argongas wird die Fab'-Lösung mit der Lösung des Kopplungsproduktes versetzt und 17 h bei Raumtemperatur inkubiert.

Das Reaktionsgemisch wird chromatographisch mittels Gelfiltration (z.B. Superdex®, Pharmacia/LKB , 150 mM KCl, 20 mM Phosphatpuffer, 1 mM EDTA, 0,02 % NaN₃, pH = 7,0) gereinigt. Die Fraktionen, die das gewünschte Produkt enthalten, werden vereinigt.

### Beispiel 2

### Turbidimetrischer Immunoassay mit anti-Transferrin Fab'-Albumin-Konjugat

### Reaktionspuffer:

0,1 M TRIS · HCl-Puffer, pH = 7,6, 6 % Polyethylenglycol 6000, 0,1 % CHAPS, 0,1 % TRITON® X-100, 0,02 % NaN₃.

### Fab'-Albumin:

hergestellt nach Beispiel 1.

### Transferrin:

Humanes Transferrin wurde in 0,1 M TRIS · HCl-Puffer, pH = 7,6 gelöst. Durch serielle Verdünnung wurden Konzentrationen im Bereich bis zu 100 mg/l Transferrin hergestellt.

### Geräteeinstellung:

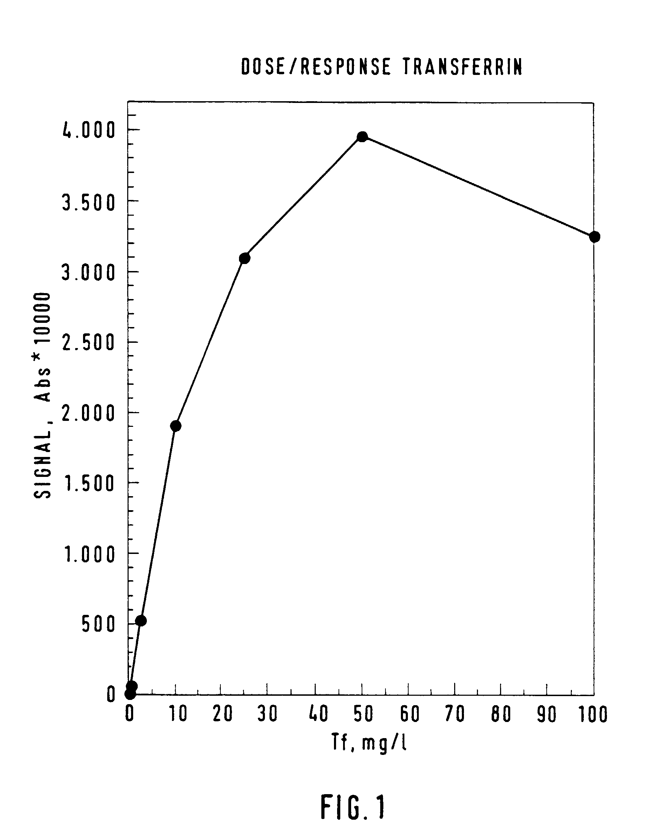

Der Test wird am klinisch-chemischen Testautomaten EPOS 5060 Analyzer als Endpunkttest durchgeführt. Zunächst werden 40 µl Probe (Transferrinlösung) und 200 µl Reaktionspuffer pipettiert, gemischt und bei 37 °C 64 sec vorinkubiert. Dann werden 30 µl Fab'-Albumin-Konjugat dazu pipettiert, gemischt und die resultierende Extinktion nach 288 sec gemessen. Der Gerätetakt wird auf 16 sec gesetzt. Die Extinktion am Ende der Vorinkubationszeit, vor Zugabe des Startreagenzes, wird zur Ermittlung des Probenleerwerts verwendet. Die erhaltene Kalibrationskurve (Extinktion/Konzentration) ist in Abb. 1 dargestellt.

### Beispiel 3

### Herstellung eines anti-CRP Fab'-Albumin-Konjugats

250 mg lyophilisiertes anti-CRP-IgG vom Schaf werden in 20 ml einer 150 mM NaCl-Lösung gelöst. Die Lösung wird mit 4 ml 1 M Natriumacetat-Puffer, pH = 4,9, versetzt. Der pH-Wert der Lösung wird mit 1 M Essigsäure auf pH = 4,35 eingestellt. 6,3 mg Pepsin werden zugegeben, gemischt und ca. 16 h bei 37 °C inkubiert. Die erhaltenen F(ab')₂-Fragmente werden chromatographisch mittels Gelfiltration (z.B. Superdex®, Pharmacia/LKB, 20 mM Kalium-phosphat-Puffer, pH = 7,0, 150 mM KCl) gereinigt. Die vereinigten Fraktionen enthalten 122 mg F(ab')₂-Fragmente. Der Pool wird auf 12 ml in einer Ultrafiltrationszelle aufkonzentriert (12,3 mg/ml F(ab')₂).

6 ml dieser Lösung (73,3 mg F(ab')₂) werden mit 1 ml 1 M Natriumacetat-Puffer, pH = 4,9, versetzt. Der pH-Wert wird mit 1 M Essigsäure auf 5,0 eingestellt. Zu dieser Lösung werden 0,35 ml einer wäßrigen 11%igen Cysteaminhydrochlorid-Lösung gegeben. Die Reaktionsmischung wird mit Argongas überschichtet und 2 h bei 30 °C inkubiert. Die entstandenen Fab'-Fragmente werden durch Gelfiltration über Sephadex® G-25 gereinigt. Es werden 53 mg Fab'-Fragmente erhalten. 14,6 mg Rinderserumalbumin werden in 1,8 ml Wasser gelöst. Zu dieser Lösung werden 7,2 mg SMCC in 0,18 ml Dioxan gegeben und 1 h bei 30 °C inkubiert. Das erhaltene BSA-SMCC-Konjugat wird gelchromatographisch über Sephadex® G-25 gereinigt. 14,3mg BSA-SMCC-Konjugat werden erhalten. Unter Argongas wird die Lösung der Fab'-Fragmente mit der BSA-SMCC-Konjugatlösung vereinigt und 18 h bei Raumtemperatur inkubiert. Anschließend werden 0,25 ml einer 11%igen Cysteaminhydrochlorid-Lösung zugegeben. Die Reaktionsmischung wird in einer Ultrafiltrationszelle auf 8 ml aufkonzentriert. Das Reaktionsgemisch wird chromatographisch mittels Gelfiltration (z.B.Superdex®, Pharmacia/LKB, 150 mM KCl, 20 mM Phosphatpuffer, 1 mM EDTA, 0,02 % NaN₃, pH = 7,0) gereinigt. Die Fraktionen, die das gewünschte Produkt enthalten, werden zusammengefaßt (21 ml).

### Beispiel 4

### Turbidimetrischer Immunoassay mit anti-CRP Fab'-Albumin-Konjugat

### Reaktionspuffer:

0,1 M TRIS · HCl-Puffer, pH = 7,6, 6 % Polyethylenglycol 6000, 0,1 % CHAPS, 0,1 % TRITON® X-100, 0,02 % NaN₃

### Fab'-Albumin:

hergestellt nach Beispiel 3

### CRP:

Humanes CRP wird in 0,1 M TRIS · HCl-Puffer, pH = 7,6, gelöst. Durch serielle Verdünnung werden Konzentrationen im Bereich bis zu 100 mg/l CRP hergestellt.

### Geräteeinstellung:

Der Test wird am klinisch-chemischen Testautomaten EPOS 5060 Analyzer als Endpunkttest durchgeführt. Zunächst werden 20 µl Probe (CRP-Lösung) und 220 µl Reaktionspuffer pipettiert, gemischt und bei 37 °C 64 sec vorinkubiert. Dann werden 30 µl Fab'-Albumin-Konjugat dazu pipettiert, gemischt und die resultierende Extinktion nach 288 sec gemessen. Der Gerätetakt wird auf 16 sec gesetzt. Die Extinktion am Ende der Vorinkubationszeit, vor Zugabe des Startreagenzes, wird zur Ermittlung des Probenleerwerts verwendet.

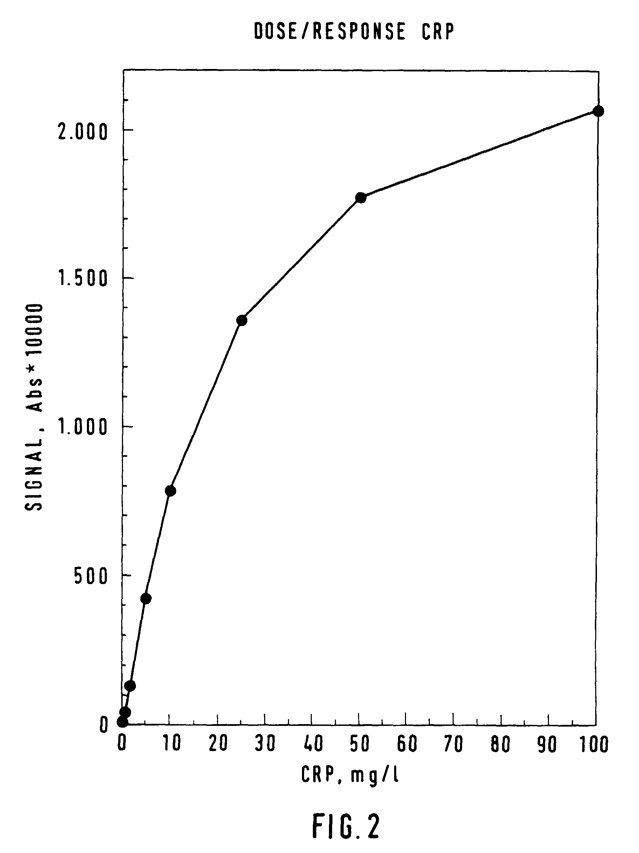

Die erhaltene Kalibrationskurve (Extinktion/Konzentration) ist in Abb. 2 dargestellt.

### Beispiel 5

### Herstellung eines anti-Transferrin Fab'-Thyroglobulin-Konjugats

Anti-Transferrin IgG (Schaf) wird analog Beispiel 1 mit Pepsin behandelt. Die F(ab')₂-Fragmente werden chromatographisch mittels Gelfiltration gereinigt. 1,3 ml einer Lösung von 41,5 mg dieser F(ab')₂-Fragmente in Phosphat-Puffer (20 mM Phosphat, 150 mM KCl, pH = 7,0) werden mit 0,2 ml 1 M Acetat-Puffer, pH = 5,0, versetzt und mit Argongas überschichtet. Es werden 0,1 ml einer 11%igen Cysteaminhydrochlorid-Lösung in entgastem Wasser zugegeben und 2 h bei 37 °C inkubiert. Die erhaltenen Fab'-Fragmente werden gelchromatographisch über Sephadex® G-25 gereinigt. Die Ausbeute beträgt 34,6 mg Fab'-Fragmente in 2 ml Elutionspuffer.

25 mg Thyroglobulin (Schwein) werden in 3 ml Phosphat-Puffer (20 mM Phosphat, 150 mM KCl, pH = 7,0) gelöst. 0,1 ml einer Lösung von 27 mg/ml SMCC in Dioxan werden zugegeben, gemischt und 30 min bei 30 °C inkubiert. Das Reaktionsgemisch wird gelchromatographisch über Sephadex® G-25 gereinigt. Zu dem Kopplungsprodukt wird portionsweise die Lösung der Fab'-Fragmente gegeben und über Nacht bei Raumtemperatur inkubiert.

Die erhaltenen Thyroglobulin-(Fab')ₙ-Konjugate werden chromatographisch mittels Gelfiltration gereinigt (150 mM KCl, 20 mM Phosphatpuffer, 1 mM EDTA, 0,02 % NaN₃, pH = 7,0). Die Fraktionen, die das gewünschte Produkt enthalten, werden zusammengefaßt (24 ml).

### Beispiel 6

### Turbidimetrischer Immunoassay mit anti-Transferrin Fab'-Thyroglobulin-Konjugat

### Reaktionspuffer:

0,1 M TRIS · HCl-Puffer, pH = 7,6, 5 % Polyethylenglycol 6000, 0,1 % CHAPS, 0,1 % TRITON® X-100, 0,02 % NaN₃

### Fab'-Thyroglobulin:

herstellt nach Beispiel 5

### Transferrin:

Humanes Transferrin wird in 0,1 M TRIS · HCl-Puffer, pH = 7,6, gelöst. Durch serielle Verdünnung werden Konzentrationen im Bereich bis zu 100 mg/l Transferrin hergestellt.

### Geräteeinstellung:

Der Test wird am EPOS 5060 Analyzer als Endpunkttest durchgeführt. Zunächst werden 15 µl Probe (Transferrin-Lösung) und 235 µl Reaktionspuffer pipettiert, gemischt und bei 37 °C 64 sec vorinkubiert. Anschließend werden 60 µl Fab'-Thyroglobulin-Konjugat dazu pipettiert, gemischt und die resultierende Extinktion nach 288 sec gemessen. Der Gerätetakt wird auf 16 sec gesetzt. Die Extinktion am Ende der Vorinkubationszeit, vor Zugabe des Startreagenzes, wird zur Ermittlung des Probenleerwerts verwendet.

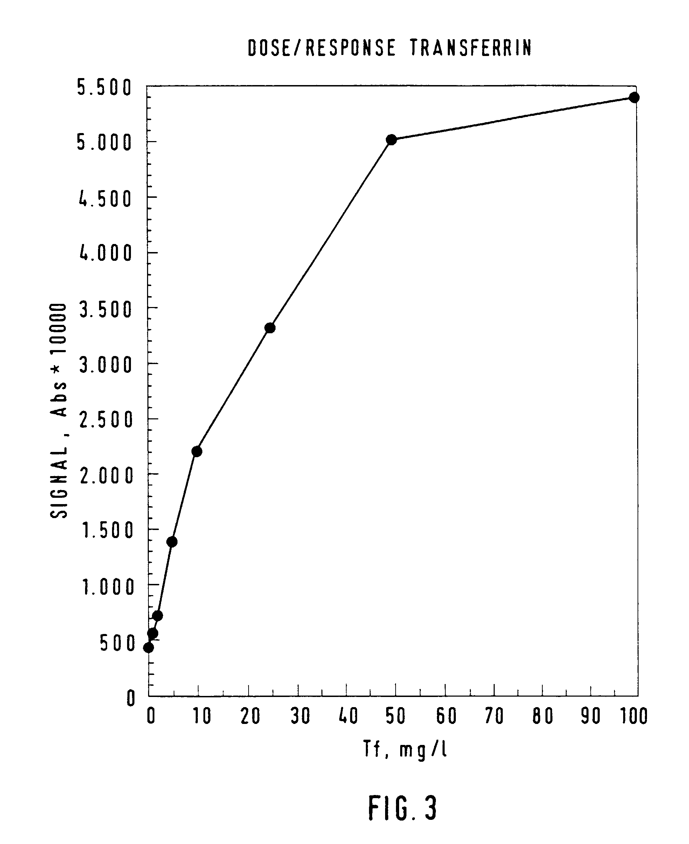

Die erhaltene Kalibrationskurve (Extinktion/Konzentration) ist in Abb. 3 dargestellt.

### Beispiel 7

### Vergleichsversuch Transferrinbestimmung

### Reaktionspuffer:

0,1 H TRIS·HCl-Puffer, pH = 7,6, 5 % Polyethylenglycol 6000, 0,1 % CHAPS, 0,1 % TRITON® X-100, 0,02 % Natriumazid

### Fab'-Albumin:

hergestellt nach Beispiel 1

### anti-Transferrin IgG:

Ausgangsmaterial von Beispiel 1

### Transferrin:

Humanes Transferrin wird in 0,1 M TRIS · HCl-Puffer, pH = 7,6, gelöst. Durch serielle Verdünnung werden Konzentrationen im Bereich bis zu 100 mg/l Transferrin hergestellt.

### Geräteeinstellung:

Der Test wird am EPOS 5060 Analyzer als Endpunkttest durchgeführt. Zunächst werden 20 µl Probe (Transferrin-Lösung) und 235 µl Reaktionspuffer pipettiert, gemischt und bei 37 °C 64 sec vorinkubiert. Anschließend werden 50 µl Fab'-Albumin-Konjugat bzw. anti-Transferrin-IgG dazu pipettiert, gemischt und die resultierende Extinktion nach 288 sec gemessen. Der Gerätetakt wird auf 16 sec gesetzt. Die Extinktion am Ende der Vorinkubationszeit, vor Zugabe des Startreagenzes, wird zur Ermittlung des Probenleerwerts verwendet.

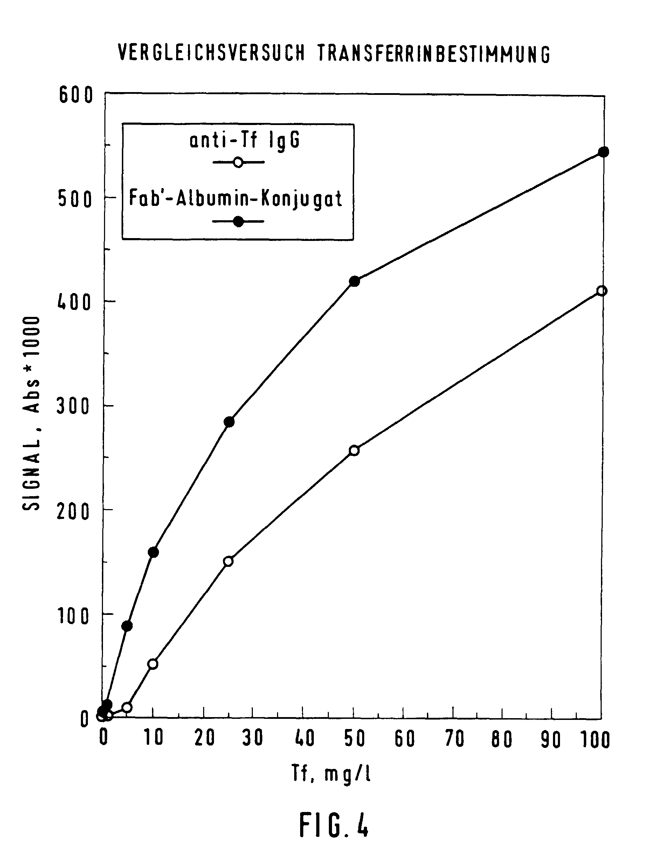

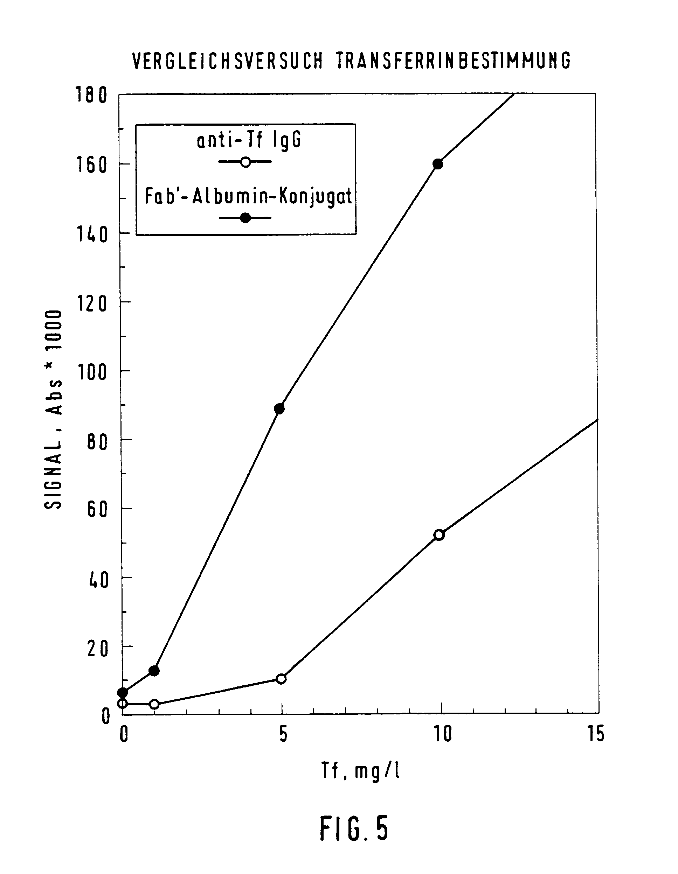

Die erhaltenen Kalibrationskurven (Extinktion/Konzentration) mit Fab'-Albumin-Konjugat bzw. anti-Transferrin-IgG sind in Abb. 4 dargestellt. Abb. 5 zeigt einen Ausschnitt dieser Kalibrationskurven für niedrige Transferrin-Konzentrationen. Im Vergleich zu nativem anti-Transferrin-IgG werden mit dem Fab'-Albumin-Konjugat unter ansonsten gleichen Bedingungen deutlich höherer Meßsignale erhalten.

### Beispiel 8

Nephelometrischer Immunoassay mit anti-CRP Fab'-Albumin-Konjugat

### Reaktionspuffer:

0,1 M TRIS·HCl-Puffer, pH = 7,6, 5 % Polyethylenglycol 6000, 0,1 % CHAPS, 0,1 % TRITON® X-100, 0,02 % Natriumazid

### Fab'-Albumin:

hergestellt nach Beispiel 3

### CRP:

Humanes CRP wird in 0,1 M TRIS HCl-Puffer, pH = 7,6, gelöst. Durch serielle Verdünnung werden Konzentrationen im Bereich bis zu 77,5 mg/l CRP hergestellt.

### Geräteeinstellungen:

Der Test wird als manueller Endpunkttest am Fluoreszenz-Spektrophotometer durchgeführt (HITACHI F 4000). Das Gerät mißt Streulicht-Intensitäten unter einem Winkel von 90°. Die Intensitäten werden als arbitrary units (a.u.) angegeben. Als Excitations- und Emissionswellenlänge werden 340 nm eingestellt.Die Spaltbreite wird auf 3 mm gesetzt. Nach vorherigem Nullabgleich wird eine Spreizung von 0-9999 a.u. gewählt.

In eine 1 cm Fluoreszenzküvette werden 200 µl Probe und 2500 µl Reaktionspuffer pipettiert, gemischt und bei Raumtemperatur 90 sec vorinkubiert. Anschließend werden 300 µl Fab'-Albumin-Konjugat dazu pipettiert, gemischt und der Anstieg der Streulicht-Intensität über 10 min verfolgt. Die erhaltenen Meßwerte werden gegen die CRP-Konzentrationen aufgetragen.

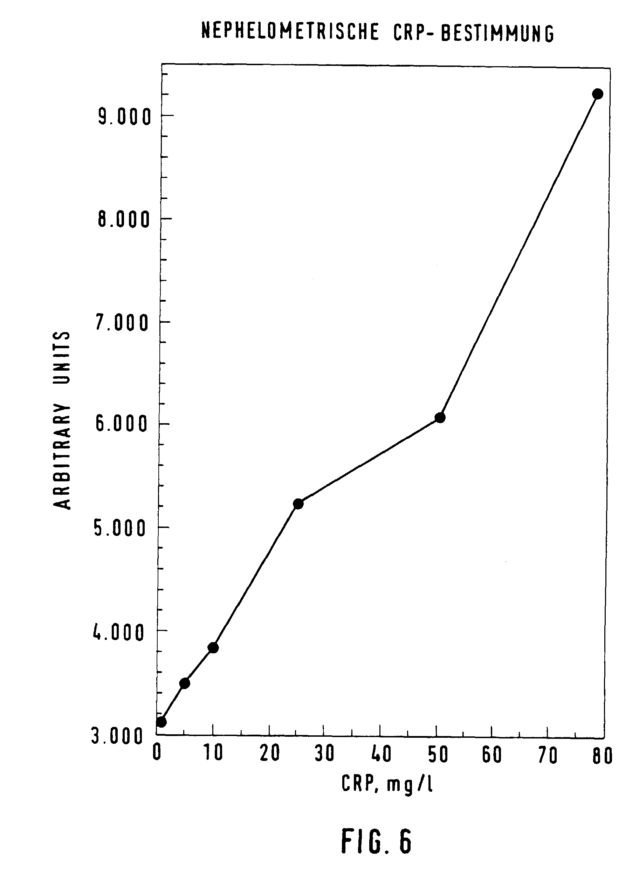

Die Kalibrationskurve (Streulicht-Intensität als a.u./Konzentration) ist in Abb. 6 dargestellt.

## Patentansprüche

1. Verfahren zur turbidimetrischen oder nephelometrischen Bestimmung von Analyten in Flüssigkeiten mit Hilfe einer Antikörper-Bindungsreaktion, dadurch gekennzeichnet, daß man ein Polypeptid mit Antikörpern oder deren Fragmenten umsetzt, das erhaltene Kopplungsprodukt mit dem Analyten inkubiert und die entstehende Trübung mißt.

2. Verfahren nach Anspruch 1, dadurch gekennzeichnet, daß man als Polypeptid ein Protein mit einem Molekulargewicht von mindestens 10 000 D verwendet.

3. Verfahren nach den Ansprüchen 1 bis 2, dadurch gekennzeichnet, daß man als Polypeptid ein globuläres Protein verwendet.

4. Verfahren nach den Ansprüchen 1 bis 3, dadurch gekennzeichnet, daß man als Polypeptid Albumin, Thyroglobulin, Transferrin, Hemocyanin, verwendet.

5. Verfahren nach den Ansprüchen 1 bis 4, dadurch gekennzeichnet, daß man als Fragmente Fab'-Fragmente verwendet.

## Claims

1. Method for the turbidimetric or nephelometric determination of analytes in liquids with the aid of an antibody-binding reaction, which is characterised in that a polypeptide is reacted with antibodies or fragments thereof, the coupling product resulting therefrom is incubated with the analyte, and the resulting turbidity is measured.

2. Method according to Claim 1, characterised in that a protein with a molecular weight of at least 10,000 D is used as polypeptide.

3. Method according to Claims 1 to 2, characterised in that a globular protein is used as polypeptide.

4. Method according to Claims 1 to 3, characterised in that albumin, thyroglobulin, transferrin, haemocyanin is used as polypeptide.

5. Method according to Claims 1 to 4, characterised in that Fab' fragments are used as fragments.

## Revendications

1. Procédé pour la détermination turbidimétrique ou néphélométrique d'analytes dans des liquides, à l'aide d'une réaction de fixation d'anticorps, caractérisé en ce que l'on fait réagir un polypeptide avec des anticorps ou des fragments de ceux-ci, on met le produit de couplage obtenu à incuber avec l'analyte et on mesure le trouble résultant.

2. Procédé selon la revendication 1, caractérisé en ce que l'on utilise comme polypeptide une protéine ayant une masse moléculaire d'au moins 10 000 Da.

3. Procédé selon la revendication 1 ou 2, caractérisé en ce que l'on utilise comme polypeptide une protéine globulaire.

4. Procédé selon les revendications 1 à 3, caractérisé en ce que l'on utilise comme polypeptide l'albumine, la thyroglobuline, la transferrine, l'hémocyanine.

5. Procédé selon les revendications 1 à 4, caractérisé en ce que l'on utilise en tant que fragments des fragments Fab'.
